# EUROPEAN PATENT SPECIFICATION

(11) **EP 1 589 826 B1**
(45) Date of publication and mention of the grant of the patent: **15.04.2009**
(21) Application number: 04707886.0
(22) Date of filing: 04.02.2004
(51) Int. Cl.: A23G 4/00, A23G 4/20

(54) **COMPRESSED CHEWING GUM TABLET**
KOMPRIMIERTE KAUGUMMITABLETTE
PASTILLE COMPRESSEE DE GOMME A MACHER

(30) Priority: 04.02.2003 WO PCT/DK03/00070; 30.12.2003 WO PCT/DK03/00941
(43) Date of publication of application: 02.11.2005
(73) Proprietor: Gumlink A/S, 7100 Vejle (DK)
(72) Inventor: NISSEN, Vibeke, DK-7000 Fredericia (DK); WITTORFF, Helle, DK-7120 Vejle Ost (DK); ANDERSEN, Lone, DK-5500 Middelfart (DK)
(74) Representative: Olesen, Kaj
(86) International application number: PCT/DK2004/000081
(87) International publication number: WO 2004/068965

(56) References cited:
- EP-A- 0 151 344
- WO-A-02/076229
- WO-A-02/076231
- WO-A-20/04004479
- DE-A- 2 808 160
- DE-A- 3 732 677
- GB-A- 1 484 832
- US-A- 4 971 806

## Description

### FIELD OF THE INVENTION

The present invention relates to an at least partly biodegradable chewing gum tablet according to claim 1.

### Background of the invention

Various ways of producing chewing gum tablets are known within the art, both with respect to the applied basic ingredients and with respect to the methods by which the final chewing gum tablets are made.

Thus, conventional chewing gum may for example be prepared by initial preparation of a gum base by mixing of water-insoluble ingredients such as elastomers and resins, typically under pressure and raised temperature. Secondly, the chewing gum ingredients, typically the water-soluble ingredients and for example flavor are added to the gum base, again by mixing. The final tablet may then be provided by a simple forming of the final chewing gum mix into the desired chewing gum tablet forms, e.g. by a kind of compacting. The above-mentioned process may be performed on a continuous basis or on a batch basis.

Such type of chewing gum is typically preferred when addressing the broad consumer market, or large-scale production, among many reasons due to the very advantageous texture of the final product. Hence, for many years this method has broadly been preferred.

An example of such chewing gum is described in US 4,847,090, in which at least one preprocessed string of final chewing gum mixture is laminated or gathered together with another layer of different compositional character.

In WO 02/076231, a chewing gum is disclosed in which the gum base comprises at least one biodegradable polymer, whereby certain rheological properties are obtained. For the preparation, processes of mixing in a mixer and forming the chewing gum into e.g. cores are disclosed.

Another method applied, which is basically very different than the above described, may broadly be described as an initial conventional mixing of the gum base, as above described followed by a granulation of the obtained gum base mix. The obtained gum base granules may then be mixed with further chewing gum ingredients, such as sweeteners and flavor. This final granules mix may then be compressed under high pressure (typically when applying cooling) into a chewing gum tablet.

This type of chewing gum, compressed chewing gum, has been widely used especially within a segment of medical chewing gum due to the thereto-related relatively careful way of handling the chewing gum ingredients and especially the active ingredient typically being quite vulnerable to for example high temperatures.

The present invention deals with the last mentioned type of chewing gum, the compressed chewing gum.

Typically, as mentioned above, compressed chewing gum has been acknowledged as quite suitable for the use of vulnerable ingredients.

One problem of the above-mentioned compressed chewing gum is that the chewing gum may be relatively expensive in manufacture and moreover, if a further processing is desired, such as coating of the final tablet, the initially gained benefits may be somewhat lost due to increased manufacturing costs and even worse, due to the stress- and temperature invoked weakening of finalizing the tablet by coating.

A further problem of the above-mentioned compressed chewing gum is that undesired interaction between chewing gum ingredients restrict the possible variations and applications offered by the technique.

A chewing gum tablet of the above-described type is disclosed in DE 28 08 160. The disclosed chewing gum tablet is obtained by compression of a chewing granulate, and the tablet may be formed by several different layers of chewing granulates mixed with different ingredients, such as sweeteners or active ingredients. A problem of the disclosed tablet is that the requirements to the mixture of the different layers are somewhat strict in the sense that all the layers are made on the basis of chewing gum granules mixed with different ingredients. In other words, chewing gum granulates must be present in a substantial amount in each layer, thereby restricting the choice of ingredients and especially the possible concentrations.

Moreover, a chewing gum composition for use in the preparation of a chewing gum tablet is disclosed in EP 0 151 344. The disclosed composition comprises gum base, grinding aid, sweetening agent, compression aid and a certain moisture content.

In US 4,161,544, a process is disclosed for making a pourable material, and a powder obtained by such process is compressed to a desired shape.

It is an object of the invention to obtain a compressed chewing gum suffering from few or none of the above-mentioned disadvantages.

### Summary of the invention

The invention relates to a chewing gum tablet (10, 20, 30, 40, 50) comprising at least two individual coherent chewing gum modules (11, 12; 21, 22, 23; 31, 32; 41, 42; 51, 52)
at least one of said chewing gum modules comprising compressed gum base granules and wherein
said compressed gum base granules comprise at least one biodegradable polyester polymer.

According to the invention, compressed gum base-comprising chewing gum granules is provided by means of compression of gum base granules, optionally added with further chewing gum ingredients or other types of gum base granules.

Thus, the resulting compressed chewing gum tablet according to the invention is basically formed by granules gathered by compression in contrast to e.g. conventional mixing of gum base pellets.

This applies in particular well to biodegradable gum base and chewing gum ingredients, which are typically vulnerable to conventional mixing.

According to the invention, different functional ingredients of the chewing gum may be not only mutually separated but also separated from the applied biodegradable gum base. Thus, the invention facilitates the use of different compounds reactive with the applied biodegradable polymers due to the fact that the biodegradable polymers may be physically separated from such compounds, i.e. comprised in different modules of the chewing gum. Thus, a mutual reaction may be completely or at least partly avoided until the chewing gum is chewed or until after the chewing gum has been chewed.

In an embodiment of the invention, a chewing gum comprising at least two different biodegradable polymers exhibits an improved texture prior to any adding of for example softeners. It has been realized that the desired chewing gum texture properties, contrary to every expectation and any prior art disclosures, may actually be obtained when combining biodegradable chewing gum polymers, for example in the gum base or in the final gum.

The fact that biodegradable polymers may actually be configured into a suitable polymer gum base, e.g. at least one biodegradable elastomer and at least one biodegradable synthetic resin substitute, facilitate the possibility of providing a completely biodegradable chewing gum.

According to an embodiment of the invention, a chewing gum should preferably comprise different biodegradable polymers in order to enable a uniform or a certain desired release profile over time.

According to an embodiment of the invention, it has been realized that chewing gum made on the basis of biodegradable polymers features an improved release of flavors, active ingredients or for example sweeteners when compared to release in chewing gums made on a conventional basis. Specifically, it has been established that different biodegradable polymers typically result in very different release properties during the complete chewing phase when compared to conventional chewing gum

Moreover, according to the invention, it has been established that the different release profiles may in fact be sort of super positioned in order to obtain a desired release profile.

According to an embodiment of the invention, a mechanically stable compressed chewing gum tablet has been obtained by applying at least one biodegradable polymer as a part of the gum base of the compressed chewing gum tablet.

Thus, experiments have shown that a compressed chewing gum tablet featuring improved mechanical stability may be obtained by applying biodegradable polymers partly or solely as the gum base forming polymer matrix.

Moreover, according to an embodiment of the invention, it has been established that a tablet formed according to the invention is relatively stable when compared to conventional compressed chewing gum tablets on the basis of non-degradable polymers prior to the initial intended chewing.

It should be stressed that although the present invention focuses primarily on a few in particular advantageous groups of biodegradable polymers, the present invention applies generally to biodegradable polymers in the sense that the surprising effect of obtaining a mechanically stable compressed tablet compared to conventionally mixed chewing gum applies generally in spite of pre-chew degradation.

In an embodiment of the invention, said gum base comprises substantially solely at least one biodegradable polymer.

According to an embodiment of the invention, a gum base is advantageously made solely on the basis of one or more biodegradable polymers. Again, experiments have shown that such gum base is advantageously applied on the basis of gum base granules.

In an embodiment of the invention, at least one of said at least two different biodegradable polymers comprises at least one biodegradable elastomer, and at least one of said at least two different biodegradable polymers comprises at least one biodegradable elastomer plasticizer, said biodegradable plasticizer comprising at least one biodegradable polymer.

According to a preferred embodiment of the invention it has been realized that it is in fact possible to pair a biodegradable polymer plasticizer with an elastomer without compromising the desire for non-tack. Moreover, it has been realized that improved texture may be obtained by incorporation of biodegradable plasticizers in a chewing gum or the gum base.

Further significant chewing gum characteristics may also be improved compared to conventional biodegradable single or dual elastomer system.

A group of elastomer plasticizers is often functionally referred to as synthetic or natural resins within the art. Therefore, according to the terminology applied for the purpose of describing the invention, the term resin may refer broadly to the elastomer plasticizing function, unless specific reference to named resin types are mentioned.

In an embodiment of the invention substantially all the chewing gum polymers are biodegradable.

In an embodiment of the invention, the applied polymers are substantially all biodegradable, thereby obtaining a chewing gum tablet, which is substantially free of non-biodegradable polymers.

In an embodiment of the invention wherein at least one of said biodegradable polymers comprises a polyester produced through reaction of at least one alcohol or derivative thereof and at least one acid or derivative thereof.

In an embodiment of the invention, wherein said alcohol derivative comprises an ester of an alcohol.

In an embodiment of the invention, wherein at least one of said biodegradable polymers comprises a polymer obtained by polymerization of a at least one cyclic ester.

In an embodiment of the invention, said chewing gum comprises at least two different polymers.

In an embodiment of the invention the chewing gum tablet comprises a gum base content of at least 5% by weight of the tablet.

In an embodiment of the invention the chewing gum tablet (10, 20, 30, 40, 50) comprises a gum base content of at least 10% by weight, preferably at least 15% by weight of the tablet.

In an embodiment of the invention the gum base content of at least one of said chewing gum modules (12, 23, 32, 42, 52), comprising compressed gum base containing chewing gum granules, is at least 15% by weight of the tablet.

In an embodiment of the invention the gum base content of at least one of said chewing gum modules (12, 23, 32, 42, 52) comprising compressed gum base-containing chewing gum granules, is at least 20% by weight of the tablet, preferably at least 25% by weight.

In an embodiment of the invention, said chewing gum comprises at least two chewing modules having different concentrations or composition of gum base.

Different release profile may be obtained by applying different types of polymers in different layers. Thus, such as profile may e.g. be obtained by applying a biodegradable polymer in at least one layer and a non-biodegradable polymer in another.

In an embodiment of the invention said chewing gum tablet comprises at least one biodegradation enhancing compound.

In an embodiment of the invention, different compounds may be added for the purpose of improving the degradation of the biodegradable polymers. A biodegradation enhancing compound may also be referred to as a polymer degrading substance.

In an embodiment of the invention said at least one biodegradation enhancing compound comprises hydrofilicity increasing compounds, preferably anhydrides or carboxylic acid compounds.

In an embodiment of the invention said at least one biodegradation enhancing compound comprises hydrolysis catalyzing compounds, preferably amino or amido compounds..

In an embodiment of the invention said at least one biodegradation enhancing compounds comprises enzymes.

In the present context the term 'enzyme' is used in the same sense as it is used within the arts of biochemistry and molecular biology. Enzymes are biological catalysts, typically proteins, but non-proteins with enzymatic properties have been discovered. Enzymes originate from living organisms where they act as catalysts and thereby regulate the rate at which chemical reactions proceed without themselves being altered in the process. The biological processes that occur within all living organisms are chemical processes, and enzymes regulate most of them. Without enzymes, many of these reactions would not take place at a perceptible rate. Enzymes catalyze all aspects of cell metabolism. This includes the conservation and transformation of chemical energy, the construction of cellular macromolecules from smaller precursors and the digestion of food, in which large nutrient molecules such as proteins, carbohydrates, and fats are broken down into smaller molecules.

Generally enzymes have valuable industrial and medical applications. The fermenting of wine, leavening of bread, curdling of cheese, and brewing of beer have been practiced from earliest times, but not until the 19th century were these reactions understood to be the result of the catalytic activity of enzymes. Since then, enzymes have assumed an increasing importance in industrial processes that involve organic chemical reactions. The investigations and developing of enzymes are still on going and new applications of enzymes are discovered. Synthetic polymers are often regarded as hardly degradable by enzymes and theories explaining this phenomenon have been proposed suggesting that enzymes tend to attack chain ends and that chain ends of man-made polymers tend to be deep in the polymer matrix. However, experiments according to the present invention surprisingly showed that the effect of adding enzymes in chewing gum apparently was that the polymers of the chewing gum experienced more degradation.

As catalysts enzymes generally may increase the rate of attainment of an equilibrium between reactants and products of chemical reactions. According to the present invention these reactants comprise polymers and different degrading molecules such as water, oxygen or other reactive substances, which may come into the vicinity of the polymers, whereas the products comprise oligomers, trimers, dimers, monomers and smaller degradation products. When reactions are enzyme catalyzed, at least one of the reactants forms a substrate for at least one enzyme, which means that a temporary binding emerges between reactants i.e. enzyme substrates and enzymes. In different ways this binding makes the reaction proceed faster, for instance by bringing the reactants into conformations or positions that favor reaction. An increase in reaction rate due to enzymatic influence i.e. catalysis generally occurs because of a lowering of an activation energy barrier for the reaction to take place. However, enzymes do not change the difference in free energy level between initial and final states of the reactants and products, as the presence of a catalyst has no effect on the position of equilibrium. When a catalytic process has been completed, the at least one enzyme releases the product or products and returns to its original state, ready for another substrate.

The temporary binding of one or more molecules of substrate happens in regions of the enzymes called the active sites and may for example comprise hydrogen bonds, ionic interactions, hydrophobic interactions or weak covalent bonds. In the complex tertiary structure of enzymes, an active site may assume the shape of a pocket or cleft, which fit particular substrates or parts of substrates. Some enzymes have a very specific mode of action, whereas others have a wide specificity and may catalyze a series of different substrates. Basically molecular conformation is important to the specificity of enzymes, and they may be rendered active or inactive by varying pH, temperature, solvent, etc. Yet some enzymes require co-enzymes or other co-factors to be present in order to be effective, in some cases forming association complexes in which a co-enzyme acts as a donor or acceptor for a specific group. Some times enzymes may be specified as endo-enzymes or exo-enzymes, thereby referring to their mode of action. According to this terminology exo-enzymes may successively attack chain ends of polymer molecules and thereby for instance liberate terminal residues or single units, whereas endo-enzymes may attack mid-chain and act on interior bonds within the polymer molecules, thereby cleaving larger molecules to smaller molecules. Generally enzymes may be attainable as liquids or powders and eventually be encapsulated in various materials.

Today, several thousand different enzymes have been discovered and more are continuously being discovered, thus the number of known enzymes is still increasing. For this reason the Nomenclature Committee of the International Union of Biochemistry and Molecular Biology (NC-IUBMB) has established a rational naming and numbering system. In the present context enzyme names are used in accordance with the recommendations devised by NC-IUBMB.

Suitable enzymes in accordance with the general principles in manufacturing an embodiment within the scope of the present invention may be identified as belonging to six classes according to their function: Oxidoreductases, transferases, hydrolases, lyases, isomerases and ligases. Oxidoreductases catalyze oxidation-reduction reactions, and the substrate oxidized is regarded as hydrogen or electron donor. Transferases catalyze transfer of functional groups from one molecule to another. Hydrolases catalyze hydrolytic cleavage of various bonds. Lyases catalyze cleavage of various bonds by other means than by hydrolysis or oxidation, meaning for example that they catalyze removal of a group from or addition of a group to a double bond, or other cleavages involving electron rearrangement. Isomerases catalyze intramolecular rearrangement, meaning changes within one molecule. Ligases catalyze reactions in which two molecules are joined.

Some preferred enzymes according to the invention are oxidoreductases, which may act on different groups of donors, such as the CH-OH group, the aldehyde or oxo group, the CH-CH group, the CH-NH₂ group, the CH-NH group, NADH or NADPH, nitrogenous compounds, a sulfur group, a heme group, diphenols and related substances, hydrogen, single donors with incorporation of molecular oxygen, paired donors with incorporation or reduction of molecular oxygen or others. Oxidoreductases may also be acting on CH₂ groups or X-H and Y-H to form an X-Y bond. Typically enzymes belonging to the group of oxidoreductases may be referred to as oxidases, oxygenases, hydrogenases, dehydrogenases, reductases or the like.

In an embodiment of the invention said biodegradation enhancing compounds are incorporated in at least one gum base containing module.

In an embodiment of the invention at least one of the chewing gum modules (11, 21, 31, 41) has a gum base content of less than 5% by weight.

In an embodiment of the invention at least one of the chewing gum modules (11, 21, 31, 41) is substantially gum base free.

In an embodiment of the invention at least two of said chewing gum modules have different plasticity or elasticity.

In an embodiment of the invention said substantially gum base free chewing gum comprises sweetener as the major ingredient

In an embodiment of the invention said chewing gum module comprising sweetener as the major ingredient forms a coating of the chewing gum tablet encapsulating the tablet completely or partly.

In an embodiment of the invention at least one of said modules comprising sweetener in the amount of at least 50% by weight.

In an embodiment of the invention at least one of said modules comprising sweetener in the amount of at least 70% by weight, preferably at least 80% by weight.

In an embodiment of the invention said biodegradation enhancing compounds are incorporated in at least one substantially gum base-free module separated from said at least one module comprising biodegradable polymers.

In an advantageous embodiment of the invention the biodegradation enhancing compounds are incorporated in one of several modules of the chewing gum tablet so that the compounds are physically separated from or at least not mixed with the applied biodegradable polymers. In this way, the biodegradation of the biodegradable polymers may be separated thereby avoiding or minimizing pre-chew degradation and increasing post-chew degradation when the chewing gum is chewed or has been chewed.

The separation of the layers may be established both by a mere separation of modules or it may be supplemented by one or further separation layers.

In an embodiment of the invention all the chewing gum modules are made by compression.

In an embodiment of the invention the chewing gum modules are gathered by means of compression.

In an embodiment of the invention at least one of the chewing gum modules are compressed when the chewing gum modules are gathered.

In an embodiment of the invention at least two, preferably all modules are compressed and gathered in one step.

In an embodiment of the invention said chewing gum modules formations having different concentrations or composition of chewing gum ingredients.

In an embodiment of the invention said chewing gum modules have different elasticity.

In an embodiment of the invention said modules are tablet slice-like layers.

In an embodiment of the invention wherein different chewing gum modules comprise ingredients intended to be separated in the tablet.

In an embodiment of the invention, mutually interacting compounds may be separated, thereby avoiding or postponing reaction between different ingredients and/or components of the chewing gum.

In an embodiment of the invention said chewing gum comprises flavoring agents.

In an embodiment of the invention said flavoring agents are comprised in modules which are substantially free of biodegradable polymers.

Flavoring agents, also referred to as aroma agents, may according to a preferred embodiment of the invention be comprised in chewing modules not-containing the biodegradable polymers, thereby avoiding an undesired pre-chew reaction between the biodegradable polymers and the flavoring agents.

Thus, it is preferred that flavoring agents are contained in a module of chewing gum not containing the biodegradable polymers, i.e. contained in one or more substantially gum-base free module or e.g. comprised in one or more conventional gum base containing modules.

In an embodiment of the invention said degradation enhancing compounds are comprised in modules which are substantially free of biodegradable polymers.

In an embodiment of the invention at least two of said chewing gum modules are separated by at least one separation layer.

In an embodiment of the invention the thickness of at least one of said substantially gum base free layers exceeds at least the smallest width of the tablet divided by 20 (twenty).

In an embodiment of the invention the thickness of at least one of said substantially gum base free layers exceeds 0.5 mm, preferably 0.7 mm.

In an embodiment of the invention said modules have different shapes.

In an embodiment of the invention said chewing gum modules are manufactured on the basis of compressible chewing gum components.

In an embodiment of the invention said chewing gum modules are manufactured on the basis of compressible chewing gum components and wherein non-compressible components are added to the compressible chewing gum components.

In an embodiment of the invention at least one chewing gum module comprises freeze-dried fruit.

In an embodiment of the invention at least one of the chewing gum modules comprises active ingredients and thereby avoiding physical or chemical interaction between the chewing gum modules of the tablet.

In an embodiment of the invention said chewing gum comprises a coating.

In an embodiment of the invention said coating comprises at least one compressed chewing gum module.

In an embodiment of the invention, the coating of the chewing may be advantageously formed by at least one compressed chewing gum module comprising sweetener as a major or sole component.

In an embodiment of the invention said gum base comprises filler in an amount of about 0% to about 50% by weight of the gum base.

In an embodiment of the invention the gum base has a water content of less than 1.0%, preferably substantially 0% by weight of the gum base.

In an embodiment of the invention the size of the gum base granules are within the range of 0.01mm · 0.01 mm to 2mm · 2mm, preferably within the range of 0.1mm ·0.1mm to 1.0 mm · 1.0mm.

In an embodiment of the invention at least one biodegradable polymer in the amount of about 1% to about 100% by weight of the gum base granules.

According to the invention it has been realized that even substantial differences in the characteristics of the different modules of the tablet may in fact be accepted, both with respect to manufacture and subsequently with respect to texture.

According to the invention, a compressed chewing gum tablet has been obtained featuring extremely impressing abilities of incorporating well-defined amounts of chewing gum ingredients combined with acceptable rheological properties of the complete tablet.

It has surprisingly been realized that multi-module compressed chewing gum may not only be produced but also inherit more than acceptable texture and mouth-feel, when the different modules are chewed into one lump comprising a mix of the remains of the different modules.

This is especially interesting when applying modules having very different nature, e.g. chewing gum based modules and sweetener modules.

### The figures

The invention will now be described with reference to the drawings of which
fig. 1a -1b illustrate a two-layer compressed tablet according to an embodiment of the invention,
fig. 2a -2b illustrate a three layer compressed tablet according to an embodiment of the invention,
fig. 3a-3b illustrate a further two layer compressed tablet according to an embodiment of the invention,
fig. 4a-4b illustrate a further two layer compressed tablet according to an embodiment of the invention,
fig. 5a-5b illustrate a further two layer compressed tablet according to an embodiment of the invention,
fig. 6a-6b illustrate a four layer compressed tablet according to an embodiment of the invention and comprising a layer of non-biodegradable gum base, and where
fig. 7a-8b illustrate three further embodiments of the invention.

### Detailed description

### COMPRESSION OF CHEWING GUM TABLETS

Chewing gum tablets are typically manufactured by applying pressure to an amount of powder by suitable compression means. Suitable compression means will be disclosed and explained below. The powder is then compressed into a compact coherent tablet.

The powder may for example comprise so-called primary particles or aggregated primary particles, also referred to as granules. When these are compressed, bonds are established between the particles or granules, thereby conferring a certain mechanical strength to the compressed tablet.

It should be noted that the above-introduced terms: powder, primary particles and granules may be somewhat misleading in the sense that the difference between primary particles and granules may very often be looked upon differently depending on the background of the user. Some may for instance regard a sweetener, such as sorbitol, as a primary particle in spite of the fact that sorbitol due to the typically preprocessing performed on sorbitol when delivered to the customer should rather be regarded as some sort of granule. The definition adopted in the description of this invention is that granules refer to macro-particles comprising more or less preprocessed primary particles. It should, however, be noted that this adoption of terms only relates to the description of background prior art and is not mandatory for defining the scope of the invention.

When pressure is applied to the powder raw material, the bulk volume is reduced and the amount of air is decreased. During this process energy is consumed. As the particles come into closer proximity to each other during the volume reduction process, bonds may be established between the particles or granules. The formation of bonds is associated with a reduction in the energy of the system as energy is released.

Volume reduction takes place by various mechanisms and different types of bonds may be established between the particles or granules depending on the pressure applied and the properties of the particles or granules.

The first thing that happens when a powder is compressed is that the particles are rearranged under low compaction pressures to form a closer packing structure. Particles with a regular shape appear to undergo rearrangement more easily than those of irregular shape. As the pressure increases, further rearrangement is prevented and subsequent volume reduction is obtained by plastic and elastic deformation and/or fragmentation of the tablet particles. Brittle particles are likely to undergo fragmentation, i.e. breakage of the original particles into smaller units. Plastic deformation is an irreversible process resulting in a permanent change of particle shape, whereas the particles resume their original shape after elastic deformation. Evidently, both plastic and elastic deformation may occur, when compressing a chewing gum tablet.

Several studies of the bond types in compressed tablets have been made over the years, typically in the context of pharmaceuticals and several techniques of obtaining compressed tablets on the basis of available powders has been provided. Such studies have been quite focused on what happens when the volume reduction is performed and how may the end-product be optimized for the given purpose. Several refinements with respect to compressed tablets has for instance been made in the addition of for example binders in the tablet raw materials for the purpose of obtaining a sufficient strength to the final compressed tablet while maintaining acceptable properties, e.g. with respect to release.

Over the years, especially the pharmaceutical industry has gradually introduced chewing gum as a mean for obtaining release of active ingredients in the oral cavity.

Traditionally, the compression technique has been preferred by the pharmaceutical industry for the manufacturing of chewing gum. As mentioned above, a problem related to the compression technique is that the nature of chewing gum granules is quite different to that of pure pharmaceutical conventional tablet powder. A further, and even more significant problem is that the required texture is basically completely different from that of a tablet intended for completely dissolving within the mouth of the user. Hence, this compression technique has been regarded as inferior with respect to the basic texture properties of therewith obtained chewing gum.

Over the last few years, however, the technique has rapidly improved especially with respect to development of gum base granulates intended for compression. Examples of such gum base granulate are described in the PCT/DK02/00461 and PCT/DK02/00462, hereby incorporated by reference.

According to the invention it has now been realized that a multi-modular chewing gum comprising a number of cohered chewing gum modules may in fact form a single piece of chewing gum having a more than acceptable texture, including the initial chew, irrespective of the fact that different modules exhibits very different properties with respect to plasticity and elasticity. Hence, even though it has be expected that for example chewing gum modules comprising sweetener, such as sorbitol as the sole or main component of that module would more or less disintegrate during the initial chew, very impressing results have been achieved.

Moreover, and again irrespective of the fact that different modules exhibit very different properties with respect to plasticity and elasticity, it has also been realized that a compressed chewing gum tablet comprising two different modules may in fact be made by compression. Hence, even though it should be expected that for example the elastic module(s) comprising gum base would affect the compression of the other layer(s) exhibiting very little elasticity, it has now been established that a final chewing gum tablet may in fact be made in one compression process, in one or several compression steps.

The gum base containing chewing modules according to the invention may typically be made on the basis of compressed gum base granulates.

The gum base granulates are made on the basis of a gum base. As used herein, the expression "gum base" refers in general to the water-insoluble part of the chewing gum which typically constitutes 10 to 90% by weight including the range of 15 -50% by weight of the total chewing gum formulation. Chewing gum base formulations typically comprise one or more elastomeric compounds which may be of synthetic or natural origin, one or more resinous compounds which may be of synthetic or natural origin, fillers, softening compounds and minor amounts of miscellaneous ingredients such as antioxidants and colorants, etc.

The composition of chewing gum base formulations, which are admixed with chewing gum ingredients as defined below, can vary substantially depending on the particular product to be prepared and on the desired masticatory and other sensory characteristics of the final product. However, typical ranges (weight%) of the above gum base components are: 5 to 50% by weight elastomeric compounds, 5 to 55% by weight elastomer plasticizers, 0 to 50% by weight filler/texturiser, 5 to 35% by weight softener and 0 to 1% by weight of miscellaneous ingredients such as antioxidants, colorants, etc.

Gum base granulates may be manufactured according to conventional methods or e.g. those described in the PCT/DK02/00461 and PCT/DK02/00462, hereby incorporated by reference.

Chewing gum ingredients.

In the present context, chewing gum ingredients include bulk sweeteners, high intensity sweeteners, flavoring agents, softeners, emulsifiers, coloring agents, binding agents, acidulants, degradation enhancing compounds, fillers, antioxidants and other components such as pharmaceutically or biologically active substances that confer desired properties to the finished chewing gum product.

Examples of suitable sweeteners are listed below.

Suitable bulk sweeteners include e.g. both sugar and non-sugar components. Bulk sweeteners typically constitute from about 5 to 95% by weight of the chewing gum, more typically about 20 to 80% by weight such as 30 to 60% by weight of the gum.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomalt, erythritol, lactitol and the like, alone or in combination.

High-intensity artificial sweetening agents can also be used alone or in combination with the above sweeteners. Preferred high-intensity sweeteners include, but are not limited to sucralose, aspartame, salts of acesulfame, alitame, saccharin and its salts, neotame, cyclamic acid and its salts, glycyrrhizin, dihydrochalcones, thaumatin, monellin, stevioside and the like, alone or in combination. In order to provide longer lasting sweetness and flavor perception, it may be desirable to encapsulate or otherwise control the release of at least a portion of the artificial sweetener. Likewise, encapsulation may be applied for the purpose of stabilizing the ingredients.

Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided e.g. using another chewing gum component, such as a resinous compound, as the encapsulation agent.

Usage level of the artificial sweetener will vary considerably depending e.g. on factors such as potency of the sweetener, rate of release, desired sweetness of the product, level and type of flavor used and cost considerations. Thus, the active level of artificial sweetener may vary from about 0.02 to 8% by weight. When carriers used for encapsulation are included, the usage level of the encapsulated sweetener will be proportionally higher. Combinations of sugar and/or non-sugar sweeteners can be used in the chewing gum formulation processed in accordance with the invention. Additionally, the softener may also provide additional sweetness such as with aqueous sugar or alditol solutions.

If a low calorie gum is desired, a low calorie bulking agent can be used. Examples of low calorie bulking agents include polydextrose, Raftilose, Raftilin, Inuline, fructooligosaccharides (NutraFlora^{®}), palatinose oligosaccharided; guar gum hydrolysates (e.g. Sun Fiber^{®}) or indigestible dextrins (e.g. Fibersol^{®}). However, other low calorie-bulking agents can be used.

Further chewing gum ingredients, which may be included in the chewing gum mixture processed in the present process, include surfactants and/or solubilisers, especially when pharmaceutically, cosmetically or biologically active ingredients are present. As examples of types of surfactants to be used as solubilisers in a chewing gum composition, according to the invention reference is made to H.P. Fiedler, Lexikon der Hilfstoffe für Pharmacie, Kosmetik und Angrenzende Gebiete, pages 63-64 (1981) and the lists of approved food emulsifiers of the individual countries. Anionic, cationic, amphoteric or non-ionic solubilisers can be used. Suitable solubilisers include lecithins, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllactylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds. The expression "solubiliser" is used in the present text to describe both possibilities; the solubiliser used must be suitable for use in food and/or medicine.

In the presence of an active ingredient the chewing gum may preferably also comprise a carrier known in the art.

One significant advantage of the present process is that the temperature throughout the entire operation can be kept at a relatively low level such as it will be described in the following. This is an advantageous feature with regard to preserving the aroma of added flavoring components, which may be prone to deterioration and/evaporation at higher temperatures. Aroma agents and flavoring agents which are useful in a chewing gum produced by the present process are e.g. natural and synthetic flavorings (including natural flavorings) in the form of freeze-dried natural vegetable components, essential oils, essences, extracts, powders, including acids and other substances capable of affecting the taste profile. Examples of liquid and powdered flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

In one preferred embodiment, the flavor is one or more natural flavoring agent(s) which is/are freeze-dried, preferably in the form of a powder, slices or pieces of combinations thereof. The particle size of such agent may be less than 3 mm, such as less than 2 mm, more preferred less than 1 mm, and calculated as the longest dimension of the particle. The natural flavoring agent may also be in a form where the particle size is from about 3 µm to 2 mm, such as from 4 µm to 1 mm. Preferred natural flavoring agents include seeds from a fruit e.g. from strawberry, blackberry and raspberry.

Various synthetic flavors, such as mixed fruit flavor may also be used according to the present invention. As indicated above, the aroma agent may be used in quantities smaller than those conventionally used. The aroma agents and/or flavors may be used in an amount from 0.01 to about 30% by weight of the final product depending on the desired intensity of the aroma and/or flavor used. Preferably, the content of aroma/flavor is in the range of from 0.2 to 3% by weight of the total composition.

According to the invention, encapsulated flavors or active ingredients, may be added to the final blend prior to compression.

Different methods of encapsulating flavors or active ingredients, which may both refer to flavors or active ingredients mixed into the gum base and flavors or active ingredients compressed into the chewing gum may e.g. include Spray drying, Spray cooling, Film coating, Coascervation, Double emulsion method (Extrusion technology) or Prilling.

Materials to be used for the above-mentioned encapsulation methods may e.g. include Gelatine, Wheat protein, Soya protein, Sodium caseinate, Caseine, Gum arabic, Mod. starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

Active ingredients may be added to chewing gum. Preferably, these ingredients should be added subsequent to any significant heating or mixing. In other words, the active ingredients should preferably be added immediately prior to the compression of the final tablet.

Referring to the process, the adding of active ingredients may be cautiously blended with pre-mixed gum base granulates and further desired ingredients, immediately prior to the final compression of the tablet.

Examples of suitable active ingredients are listed below.

In one embodiment the chewing gum according to the invention comprises a pharmaceutically, cosmetically or biologically active substance. Examples of such active substances, a comprehensive list of which is found e.g. in WO 00/25598, which is incorporated herein by reference, include drugs, dietary supplements, antiseptic agents, pH adjusting agents, anti-smoking agents and substances for the care or treatment of the oral cavity and the teeth such as hydrogen peroxide and compounds capable of releasing urea during chewing. Examples of useful active substances in the form of antiseptics include salts and derivatives of guanidine and biguanidine (for instance chlorhexidine diacetate) and the following types of substances with limited water-solubility: quaternary ammonium compounds (e.g. ceramine, chloroxylenol, crystal violet, chloramine), aldehydes (e.g. paraformaldehyde), derivatives of dequaline, polynoxyline, phenols (e.g. thymol, p-chlorophenol, cresol), hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. also Martindale, The Extra Pharmacopoeia, 28th edition, pages 547-578; metal salts, complexes and compounds with limited water-solubility, such as aluminum salts, (for instance aluminum potassium sulphate AlK(SO₄)₂,12H₂O) and salts, complexes and compounds of boron, barium, strontium, iron, calcium, zinc, (zinc acetate, zinc chloride, zinc gluconate), copper (copper chloride, copper sulphate), lead, silver, magnesium, sodium, potassium, lithium, molybdenum, vanadium should be included; other compositions for the care of mouth and teeth: for instance; salts, complexes and compounds containing fluorine (such as sodium fluoride, sodium monofluorophosphate, aminofluorides, stannous fluoride), phosphates, carbonates and selenium. Further active substances can be found in J. Dent. Res. Vol. 28 No. 2, pages 160-171,1949.

Examples of active substances in the form of agents adjusting the pH in the oral cavity include: acids, such as adipic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulphates or oxides of sodium, potassium, ammonium, magnesium or calcium, especially magnesium and calcium.

Active ingredients may comprise the below mentioned compounds or derivates thereof but are not limited thereto: Acetaminophen, Acetylsalicylsyre Buprenorphine Bromhexin Celcoxib Codeine, Diphenhydramin, Diclofenac, Etoricoxib, Ibuprofen, Indometacin, Ketoprofen, Lumiracoxib, Morphine, Naproxen, Oxycodon, Parecoxib, Piroxicam, Pseudoefedrin, Rofecoxib, Tenoxicam, Tramadol, Valdecoxib, Calciumcarbonat, Magaldrate, Disulfiram, Bupropion, Nicotine, Azithromycin, Clarithromycin, Clotrimazole, Erythromycin, Tetracycline, Granisetron, Ondansetron, Prometazin, Tropisetron, Brompheniramine, Ceterizin, leco-Ceterizin, Chlorcyclizine, Chlorpheniramin, Chlorpheniramin, Difenhydramine, Doxylamine, Fenofenadin, Guaifenesin, Loratidin, des-Loratidin, Phenyltoloxamine, Promethazin, Pyridamine, Terfenadin, Troxerutin, Methyldopa, Methylphenidate, Benzalcon. Chloride, Benzeth. Chloride, Cetylpyrid. Chloride, Chlorhexidine, Ecabet-sodium, Haloperidol, Allopurinol, Colchinine, Theophylline, Propanolol, Prednisolone, Prednisone, Fluoride, Urea, Actot, Glibenclamide, Glipizide, Metformin, Miglitol, Repaglinide, Rosiglitazone, Apomorfin, Cialis, Sildenafil, Vardenafil, Diphenoxylate, Simethicone, Cimetidine, Famotidine, Ranitidine, Ratinidine, cetrizin, Loratadine, Aspirin, Benzocaine, Dextrometorphan, Phenylpropanolamine, Pseudoephedrine, Cisapride, Domperidone, Metoclopramide, Acyclovir, Dioctylsulfosucc., Phenolphtalein, Almotriptan, Eletriptan, Ergotamine, Migea, Naratriptan, Rizatriptan, Sumatriptan, Zolmitriptan, Aluminum salts, Calcium salts, Ferro salts, Ag-salts, Zinc-salts, Amphotericin B, Chlorhexidine, Miconazole, Triamcinolonacetonid, Melatonine, Phenobarbitol, Caffeine, Benzodiazepiner, Hydroxyzine, Meprobamate, Phenothiazine, Buclizine, Brometazine, Cinnarizine, Cyclizine, Difenhydramine, Dimenhydrinate, Buflomedil, Amphetamine, Caffeine, Ephedrine, Orlistat, Phenylephedrine, Phenylpropanolamin, Pseudoephedrine, Sibutramin, Ketoconazole, Nitroglycerin, Nystatin, Progesterone, Testosterone, Vitamin B12, Vitamin C, Vitamin A, Vitamin D, Vitamin E, Pilocarpin, Aluminumaminoacetat, Cimetidine, Esomeprazole, Famotidine, Lansoprazole, Magnesiumoxide, Nizatide and or Ratinidine.

The invention is suitable for increased or accelerated release of active agents selected among the group of dietary supplements, oral and dental compositions, antiseptic agents, pH adjusting agents, anti-smoking agents, sweeteners, flavorings, aroma agents or drugs. Some of those will be described below.

The active agents to be used in connection with the present invention may be any substance desired to be released from the chewing gum. The active agents, for which a controlled and/or accelerated rate of release is desired, are primarily substances with a limited water-solubility, typically below 10 g/100 ml inclusive of substances which are totally water-insoluble. Examples are medicines, dietary supplements, oral compositions, anti-smoking agents, highly potent sweeteners, pH adjusting agents, flavorings etc.

Other active ingredients are, for instance, paracetamol, benzocaine, cinnarizine, menthol, carvone, caffeine, chlorhexidine-di-acetate, cyclizine hydrochloride, 1,8-cineol, nandrolone, miconazole, mystatine, sodium fluoride, nicotine, cetylpyridinium chloride, other quaternary ammonium compounds, vitamin E, vitamin A, vitamin D, glibenclamide or derivatives thereof, progesterone, acetylsalicylic acid, dimenhydrinate, cyclizine, metronidazole, sodium hydrogen carbonate, the active components from ginkgo, the active components from propolis, the active components from ginseng, methadone, oil of peppermint, salicylamide, hydrocortisone or astemizole.

Examples of active agents in the form of dietary supplements are for instance salts and compounds having the nutritive effect of vitamin B2 (riboflavin), B12, folinic acid, folic acid, niacine, biotine, poorly soluble glycerophosphates, amino acids, the vitamins A, D, E and K, minerals in the form of salts, complexes and compounds containing calcium, phosphorus, magnesium, iron, zinc, copper, iodine, manganese, chromium, selenium, molybdenum, potassium, sodium or cobalt.

Furthermore, reference is made to lists of nutritionists accepted by the authorities in different countries such as for instance US code of Federal Regulations, Title 21, Section 182.5013.182 5997 and 182.8013-182.8997.

Examples of active agents in the form of compounds for the care or treatment of the oral cavity and the teeth are for instance bound hydrogen peroxide and compounds capable of releasing urea during chewing.

Examples of active agents in the form of antiseptics are for instance salts and compounds of guanidine and biguanidine (for instance chlorhexidine diacetate) and the following types of substances with limited water-solubility: quaternary ammonium compounds (for instance ceramine, chloroxylenol, crystal violet, chloramine), aldehydes (for instance paraformaldehyde), compounds of dequaline, polynoxyline, phenols (for instance thymol, para chlorophenol, cresol) hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. furthermore Martindale, The Extra Pharmacopoeia, 28th edition, pages 547-578; metal salts, complexes and compounds with limited water-solubility, such as aluminum salts, (for instance aluminum potassium sulphate AlK(SO₄)₂,12H₂O) and furthermore salts, complexes and compounds of boron, barium, strontium, iron, calcium, zinc, (zinc acetate, zinc chloride, zinc gluconate), copper (copper chloride, copper sulfate), lead, silver, magnesium, sodium, potassium, lithium, molybdenum, vanadium should be included; other compositions for the care of mouth and teeth: for instance; salts, complexes and compounds containing fluorine (such as sodium fluoride, sodiummonofluorophosphate, amino fluorides, stannous fluoride), phosphates, carbonates and selenium.

Cf. furthermore J. Dent.Res. Vol. 28 No. 2, pages 160-171, 1949, wherein a wide range of tested compounds is mentioned.

Examples of active agents in the form of agents adjusting the pH in the oral cavity include for instance: acceptable acids, such as adipic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulfates or oxides of sodium, potassium, ammonium, magnesium or calcium, especially magnesium and calcium.

Examples of active agents in the form of anti-smoking agents include for instance: nicotine, tobacco powder or silver salts, for instance silver acetate, silver carbonate and silver nitrate.

In a further embodiment, the sucrose fatty acid esters may also be utilized for increased release of sweeteners including for instance the so-called highly potent sweeteners, such as for instance saccharin, cyclamate, aspartame, thaumatin, dihydrocalcones, stevioside, glycyrrhizin or salts or compounds thereof. For increased released of sweetener, the sucrose fatty acids preferable have a content of palmitate of at least 40% such as at least 50%.

Further examples of active agents are medicines of any type.

Examples of active agents in the form of medicines include caffeine, salicylic acid, salicyl amide and related substances (acetylsalicylic acid, choline salicylate, magnesium salicylate, sodium salicylate), paracetamol, salts of pentazocine (pentazocine hydrochloride and pentazocinelactate), buprenorphine hydrochloride, codeine hydrochloride and codeine phosphate, morphine and morphine salts (hydrochloride, sulfate, tartrate), methadone hydrochloride, ketobemidone and salts of ketobemidone (hydrochloride), beta-blockers, (propranolol), calcium antagonists, verapamil hydrochloride, nifedinpine as well as suitable substances and salts thereof mentioned in Pharm. Int., Nov.85, pages 267-271, Barney H. Hunter and Robert L. Talbert, nitroglycerine, erythrityl tetranitrate, strychnine and salts thereof, lidocaine, tetracaine hydrochloride, etorphine hydrochloride, atropine, insulin, enzymes (for instance papain, trypsin, amyloglucosidase, glucoseoxidase, streptokinase, streptodornase, dextranase, alpha amylase), polypeptides (oxytocin, gonadorelin, (LH.RH), desmopressin acetate (DDAVP), isoxsuprine hydrochloride, ergotamine compounds, chloroquine (phosphate, sulfate), isosorbide, demoxytocin, heparin.

Other active ingredients include beta-lupeol, Letigen^{®}, Sildenafil citrate and derivatives thereof.

Dental products include Carbamide, CPP Caseine Phospho Peptide; Chlorhexidine, Chlorhexidine di acetate, Chlorhexidine Chloride, Chlorhexidine di gluconate, Hexetedine, Strontium chloride, Potassium Chloride, Sodium bicarbonate, Sodium carbonate, Fluor containing ingredients, Fluorides, Sodium fluoride, Aluminum fluoride.

Ammonium fluoride, Calcium fluoride, Stannous fluoride, Other fluor containing ingredients Ammonium fluorosilicate, Potassium fluorosilicate, Sodium fluorosilicate, Ammonium monofluorphosphate, Calcium monofluorphosphate, Potassium monofluorphosphate, Sodium monofluorphosphate, Octadecentyl Ammonium fluoride, Stearyl Trihydroxyethyl Propylenediamine Dihydrofluoride,

Vitamins include A, B1, B2, B6, B12, Folinic acid, Folic acid, niacin, Pantothensyre, biotine, C, D, E, K. Minerals include Calcium, phosphor, magnesium, iron, Zinc, Cupper, Iod, Mangan, Crom, Selene, Molybden. Other active ingredients include: Q10^{®}, enzymes. Natural drugs including Ginkgo Biloba, ginger, and fish oil.
The invention also relates to use of migraine drugs such as Serotonin antagonists: Sumatriptan, Zolmitriptan, Naratriptan, Rizatriptan, Eletriptan; nausea drugs such as Cyclizin, Cinnarizin, Dimenhydramin, Difenhydrinat; hay fever drugs such as Cetrizin, Loratidin, pain relief drugs such as Buprenorfin, Tramadol, oral disease drugs such as Miconazol, Amphotericin B, Triamcinolonaceton; and the drugs Cisaprid, Domperidon, Metoclopramid. In a preferred embodiment the invention relates to the release of Nicotine and its salts.

Above mentioned active ingredients and/or flavors may be pre-mixed into the gum base or of course added to the non-or low CG incorporated layer.

When the gum base granules comprises pre-mixed active ingredients, a controlled release of active ingredients may be obtained by means of at least a double active ingredients buffer. The first buffer comprising active ingredients blended into the final mix immediately prior to compression and the second buffer comprising active ingredients blended into the gum base prior to the blending of gum base and gum base ingredients.

In accordance with the invention, the chewing gum element comprises about 0 to about 75% by weight of an outer coating applied onto the chewing gum center. In the present context, a suitable outer coating is any coating that results in extended storage stability of the compressed chewing gum products as defined above, relative to a chewing gum of the same composition that is not coated. Thus, suitable coating types include hard coatings, film coatings and soft coatings of any composition including those currently used in coating of chewing gum, pharmaceutical products and confectioneries.

According to a preferred embodiment of the invention, film coating is applied to the compressed chewing gum tablet.

One presently preferred outer coating type is a hard coating, which term is used in the conventional meaning of that term including sugar coatings and sugar-free (or sugarless) coatings and combinations thereof. The object of hard coating is to obtain a sweet, crunchy layer which is appreciated by the consumer and to protect the gum centers for various reasons as. In a typical process of providing the chewing gum centers with a protective sugar coating the gum centers are successively treated in suitable coating equipment with aqueous solutions of crystallisable sugar such as sucrose or dextrose, which, depending on the stage of coating reached, may contain other functional ingredients, e.g. fillers, colors, etc. In the present context, the sugar coating may contain further functional or active compounds including flavor compounds, pharmaceutically active compounds and/or polymer degrading substances.

In the production of chewing gum it may, however, be preferred to replace the cariogenic sugar compounds in the coating by other, preferably crystallisable, sweetening compounds that do not have a cariogenic effect. In the art such coatings are generally referred to as sugarless or sugar-free coatings. Presently preferred non-cariogenic hard coating substances include polyols, e.g. sorbitol, maltitol, mannitol, xylitol, erythritol, lactitol, isomalt and tagatose which are obtained by industrial methods by hydrogenation of D-glucose, maltose, fructose or levulose, xylose, erythrose, lactose, isomaltulose and D-galactose, respectively.

In a typical hard coating process, as it will be described in details in the following, syrup containing crystallisable sugar and/or polyol is applied onto the gum centers and the water it contains is evaporated off by blowing with warm, dry air. This cycle must be repeated several times, typically 10 to 80 times, in order to reach the swelling required. The term "swelling" refers to the increase in weight of the products, as considered at the end of the coating operation by comparison with the beginning, and in relation to the final weight of the coated products. In accordance with the present invention, the coating layer constitutes for example about 0 to 75% by weight of the finished chewing gum element, such as about 10 to 60% by weight, including about 15 to 50% by weight.

In further useful embodiments the outer coating of the chewing gum element of the invention is an element that is subjected to a film coating process and which therefore comprises one or more film-forming polymeric agents and optionally one or more auxiliary compounds, e.g. plasticizers, pigments and opacifiers. A film coating is a thin polymer-based coating applied to a chewing gum center of any of the above forms. The thickness of such a coating is usually between 20 and 100 µm. Generally, the film coating is obtained by passing the chewing gum centers through a spray zone with atomized droplets of the coating materials in a suitable aqueous or organic solvent vehicle, after which the material adhering to the gum centers is dried before the next portion of coating is received. This cycle is repeated until the coating is complete.

In the present context, suitable film-coating polymers include edible cellulose derivatives such as cellulose ethers including methylcellulose (MC), hydroxyethyl cellulose (HEC), hydroxypropyl cellulose (HPC) and hydroxypropyl methylcellulose (HPMC). Other useful film-coating agents are acrylic polymers and copolymers, e.g. methylacrylate aminoester copolymer or mixtures of cellulose derivatives and acrylic polymers. A particular group of film-coating polymers also referred to as functional polymers are polymers that, in addition to its film-forming characteristics, confer a modified release performance with respect to active components of the chewing gum formulation. Such release modifying polymers include methylacrylate ester copolymers, ethylcellulose (EC) and enteric polymers designed to resist the acidic stomach environment, yet dissolve readily in the duodenum. The latter group of polymers includes: cellulose acetate phthalate (CAP), polyvinyl acetate phthalate (PVAP), shellac, metacrylic acid copolymers, cellulose acetate trimellitate (CAT) and HPMC. It will be appreciated that the outer film coating according to the present invention may comprise any combination of the above film-coating polymers.

In other embodiments, the film-coating layer of the chewing gum elements According to the invention comprises a plasticizing agent having the capacity to alter the physical properties of a polymer to render it more useful in performing its function as a film-forming material. In general, the effect of plasticizers will be to make the polymer softer and more pliable as the plasticizer molecules interpose themselves between the individual polymer strands thus breaking down polymer-polymer interactions. Most plasticizers used in film coating are either amorphous or have very little crystallinity. In the present context, suitable plasticizers include polyols such as glycerol, propylene glycol, polyethylene glycol, e.g. the 200-6000 grades hereof, organic esters such as phthalate esters, dibutyl sebacate, citrate esters and triacetin, oils/glycerides including castor oil, acetylated monoglycerides and fractionated coconut oil.

The choice of film-forming polymer(s) and plasticizing agent(s) for an optional outer coating of the present chewing gum element is made with due consideration for achieving the best possible barrier properties of the coating in respect of dissolution and diffusion across the film of moisture and gasses.

The film coating of the chewing gum elements may also contain one or more colorants or opacifiers. In addition to providing a desired color hue, such agents may contribute to protecting the compressed gum base against pre-chewing reactions, in particular by forming a barrier against moisture and gasses. Suitable colorants/pacifiers include organic dyes and their lakes, inorganic coloring agents, e.g. titanium oxide and natural colors such as e.g. β-carotene.

Additionally, film coatings may contain one or several auxiliary substances such as flavors and waxes or saccharide compounds such as polydextrose, dextrins including maltodextrin, lactose, modified starch, a protein such as gelatine or zein, a vegetable gum and any combination thereof.

It is also an aspect of the present invention that the outer coating of the chewing gum element can contain one or more pharmaceutically or cosmetically components including those mentioned hereinbefore.

Accordingly, in further embodiments, a above hard-coated or film-coated chewing gum element of the invention is an element where the outer coating comprises at least one additive component selected from a binding agent, a moisture absorbing component, a film forming agent, a dispersing agent, an antisticking component, a bulking agent, a flavoring agent, a coloring agent, a pharmaceutically or cosmetically active component, a lipid component, a wax component, a sugar and an acid. If it is desired to defer the effect of any of these additive components in the outer coating until mastication of the chewing gum, such components may, in accordance with the invention be encapsulated using any conventional encapsulation agent such as e.g. a protein including gelatine and soy protein, a cellulose derivative including any of those mentioned above, a starch derivative, edible synthetic polymers and lipid substances, the latter optionally in the form of liposome encapsulation.

In other embodiments, the chewing gum element according to the invention is provided with an outer coating in the form generally described in the art as a soft coating. Such soft coatings are applied using conventional methods and may advantageously consist of a mixture of a sugar or any of the above non-cariogenic, sugar-less sweetening compounds, and a starch hydrolysate.

Again, it should be noted that the above-described coating is optional or that it may be postponed until it fits into the last part of the manufacturing process due to the fact that the applied barrier layer is also acting as a complete or at least a partial barrier to transfer of humidity from the environment into the tablet.

Moreover, the chewing gum tablet according to a preferred embodiment of the invention may comprise a coating mainly comprising sweeteners and made by compression.

Elastomeric compounds and elastomer plasticizers of the gum base are preferably completely or at least partly biodegradable according to the invention.

Thus, according to the invention, particularly interesting elastomeric or resinous polymer compounds comprises biodegradable polymers which, in contrast to typically used elastomers and resins, can be degraded physically, chemically or enzymatically in the environment after use of the chewing gum, thereby giving rise to less environmental pollution than chewing gums based on non-degradable polymers, as the used degradable chewing gum remnants will eventually disintegrate and/or can be removed more readily by physical or chemical means from the site where it has been dumped.

Suitable biodegradable polymers according to the present invention may be referred to as polyesters of type 1 and 2, which are described in PCT/DK03/00626, hereby incorporated by reference. Further descriptions of biodegradable polymers are disclosed in PCT/DK02/00201, PCT/DK02/00203, PCT/DK02/00205, PCT/DK02/ 00628 and PCT/DK03/00941, hereby incorporated by reference.

In the present context, the terms environmentally or biologically degradable polymer compounds refer to chewing gum base components which, after dumping the chewing gum, are capable of undergoing a physical, chemical and/or biological degradation whereby the dumped chewing gum waste becomes more readily removable from the site of dumping or is eventually disintegrated to lumps or particles which are no longer recognizable as being chewing gum remnants. The degradation or disintegration of such degradable polymers can be effected or induced by physical factors such as temperature, light, moisture, by chemical factors such as hydrolysis caused by a change in pH or by the action of enzymes capable of degrading the polymers. In other useful embodiments all of the polymer components of the gum base are environmentally degradable or biodegradable polymers.

The above-described polyester type 1 polymers may generally within the scope of the invention be prepared by step-growth polymerization of di-, tri- or higher-functional alcohols or esters thereof with di-, tri- or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed. Use of branched monomers suppresses the crystallinity of the polyester polymers. Mixing of dissimilar monomer units along the chain also suppresses crystallinity. To control the reaction and the molecular weight of the resulting polymer it is possible to stop the polymer chains by addition of monofunctional alcohols or acids and/or to utilize a stoichiometric imbalance between acid groups and alcohol groups or derivatives of either. Also the adding of long chain aliphatic carboxylic acids or aromatic monocarboxylic acids may be used to control the degree of branching in the polymer and conversely multifunctional monomers are sometimes used to create branching. Moreover, following the polymerization monofunctional compounds may be used to end cap the free hydroxyl and carboxyl groups.

In general, polyfunctional carboxylic acids are high-melting solids that have very limited solubility in the polycondensation reaction medium. Often esters or anhydrides of the polyfunctional carboxylic acids are used to overcome this limitation. Polycondensations involving carboxylic acids or anhydrides produce water as the condensate, which requires high temperatures to be driven off. Thus, polycondensations involving transesterification of the ester of a polyfunctional acid are often the preferred process. For example, the dimethyl ester of terephthalic acid may be used instead of terephthalic acid itself. In this case, methanol rather than water is condensed, and the former can be driven off more easily than water. Usually, the reaction is carried out in the bulk (no solvent) and high temperatures and vacuum are used to remove the by-product and drive the reaction to completion. In addition to an ester or anhydride, a halide of the carboxylic acid may also be used under certain circumstances.

Usually, for preparation of polyesters type 1 the preferred polyfunctional carboxylic acids or derivatives thereof are either saturated or unsaturated aliphatic or aromatic and contain 2 to 100 carbon atoms and more preferably 4 to 18 carbon atoms. In the polymerization of polyester type 1 some applicable examples of carboxylic acids, which may be employed as such or as derivatives thereof, includes aliphatic polyfunctional carboxylic acids such as oxalic, malonic, citric, succinic, malic, tartaric, fumaric, maleic, glutaric, glutamic, adipic, glucaric, pimelic, suberic, azelaic, sebacic, dodecanedioic acid, etc. and cyclic aliphatic polyfunctional carboxylic acids such as cyclopropane dicarboxylic acid, cyclobutane dicarboxylic acid, cyclohexane dicarboxylic acid, etc. and aromatic polyfunctional carboxylic acids such as terephthalic, isophthalic, phthalic, trimellitic, pyromellitic and naphthalene 1,4-, 2,3-, 2,6-dicarboxylic acids and the like. For the purpose of illustration and not limitation, some examples of carboxylic acid derivatives include hydroxy acids such as 3-hydroxy propionic acid and 6-hydroxycaproic acid and anhydrides, halides or esters of acids, for example dimethyl or diethyl esters, corresponding to the already mentioned acids, which means esters such as dimethyl or diethyl oxalate, malonate, succinate, fumarate, maleate, glutarate, adipate, pimelate, suberate, azelate, sebacate, dodecanedioate, terephthalate, isophthalate, phthalate, etc. Generally speaking, methyl esters are sometimes more preferred than ethyl esters due to the fact that higher boiling alcohols are more difficult to remove than lower boiling alcohols.

Furthermore, the usually preferred polyfunctional alcohols contain 2 to 100 carbon atoms as for instance polyglycols and polyglycerols. In the polymerization process of polyester type 1 some applicable examples of alcohols, which may be employed as such or as derivatives thereof, includes polyols such as ethylene glycol, 1,2-propanediol, 1,3-propanediol, 1,3-butanediol, 1,4-butanediol, 1,6-hexanediol, diethylene glycol, 1,4-cyclohexanediol, 1,4-cyclohexanedimethanol, neopentyl glycol, glycerol, trimethylolpropane, pentaerythritol, sorbitol, mannitol, etc. For the purpose of illustration and not limitation, some examples of alcohol derivatives include triacetin, glycerol palmitate, glycerol sebacate, glycerol adipate, tripropionin, etc.

Additionally, with regard to polyester type 1 polymerization the chain-stoppers sometimes used are monofunctional compounds. They are preferably either monohydroxy alcohols containing 1-20 carbon atoms or monocarboxylic acids containing 2-26 carbon atoms. General examples are medium or long-chain fatty alcohols or acids, and specific examples include monohydroxy alcohols such as methanol, ethanol, butanol, hexanol, octanol, etc. and lauryl alcohol, myristyl alcohol, cetyl alcohol, stearyl alcohol, stearic alcohol, etc. and monocarboxylic acids such as acetic, lauric, myristic, palmitic, stearic, arachidic, cerotic, dodecylenic, palmitoleic, oleic, linoleic, linolenic, erucic, benzoic, naphthoic acids and substituted napthoic acids, 1-methyl-2 naphthoic acid and 2-isopropyl-1-naphthoic acid, etc.

Typically, an acid catalyst or a transesterification catalyst is used in the polyester type 1 polymerization and non-limiting examples of those are the metal catalysts such as acetates of manganese, zinc, calcium, cobalt or magnesium, and antimony(III)oxide, germanium oxide or halide and tetraalkoxygermanium, titanium alkoxide, zinc or aluminum salts.

The above described polyester type 2 polymers may generally be obtained by ring-opening polymerization of one or more cyclic esters, which includes glycolides, lactides, lactones and carbonates. The polymerization process may take place in the presence of at least one appropriate catalyst such as metal catalysts, of which stannous octoate is a non-limiting example and the polymerization process may be initiated by initiators such as polyols, polyamines or other molecules with multiple hydroxyl or other reactive groups and mixtures thereof.

In an embodiment of the invention, said polyester obtained by polymerization of at least one cyclic ester is at least partly derived from α-hydroxy acids such as lactic and glycolic acids.

According to an embodiment of the invention at least one of the applied polyester polymers are derived from α-hydroxy acids such as lactic and glycolic acids. The obtained ester linkages, due to their chemical structure, are very susceptible to hydrolysis, and because these acids are natural metabolites, their esters are susceptible to a large variety of enzymatic degradation mechanisms, by the human body, animal bodies and bacteria.

In an embodiment of the invention, said polyester obtained by polymerization of at least one cyclic ester is at least partly derived from α-hydroxy acids and where the obtained polyester comprises at least 20 mole% α-hydroxy acids units, preferably at least 50 mole% α-hydroxy acids units and most preferably at least 80 mole% α-hydroxy acids units

According to a preferred embodiment of the invention, the chewing gum comprises an elastomer plasticizer comprising more then 90 mole% α-hydroxy acids, e.g. lactic acid units.

In an embodiment of the invention, at least two or more cyclic esters are selected from the groups of glycolides, lactides, lactones, cyclic carbonates or mixtures thereof.

In an embodiment of the invention, lactone monomers are chosen from the group of ε-caprolactone, δ-valerolactone, γ-butyrolactone, and β-propiolactone. It also includes ε-caprolactones, δ-valerolactones, γ-butyrolactones, or β-propiolactones that have been substituted with one or more alkyl or aryl substituents at any non-carbonyl carbon atoms along the ring, including compounds in which two substituents are contained on the same carbon atom.

In an embodiment of the invention carbonate monomer is selected from the group of trimethylene carbonate, 5-alkyl-1,3-dioxan-2-one, 5,5-dialkyl-1,3-dioxan-2-one, or 5-alkyl-5-alkyloxycarbonyl-1,3-dioxan-2-one, ethylene carbonate, 3-ethyl-3-hydroxyethyl, propylene carbonate, trimethylolpropane monocarbonate, 4, 6dimethyl-1, 3-propylene carbonate, 2, 2-dimethyl trimethylene carbonate, and 1, 3-dioxepan-2-one and mixtures thereof.

In an embodiment of the invention, cyclic ester polymers and their copolymers resulting from the polymerization of cyclic ester monomers include, but are not limited to: poly (L-lactide) ; poly (D-lactide) ; poly (D, L-lactide) ; poly (mesolactide) ; poly (glycolide) ; poly (trimethylenecarbonate) ; poly (epsilon-caprolactone) ; poly (L-lactide-co-D, L-lactide) ; poly (L-lactide-co-meso-lactide) ; poly (L-lactide-co-glycolide) ; poly (L-lactide-co-trimethylenecarbonate) ; poly (L-lactide-co-epsilon-caprolactone) ; poly (D, L-lactide-co-meso-lactide) ; poly (D, L-lactide-co-glycolide) ; poly (D, L-lactide-co-trimethylenecarbonate) ; poly (D, L-lactide-co-epsilon-caprolactone) ; poly (meso-lactide-co-glycolide) ; poly (meso-lactide-co-trimethylenecarbonate) ; poly (meso-lactide-co-epsilon-caprolactone) ; poly (glycolide-cotrimethylenecarbonate) ; poly (glycolide-co-epsilon-caprolactone).

In an embodiment of the invention, said polyester obtained by polymerization of at least one cyclic ester has a PD of 1.1 to 15, preferably 1.3 to 9.

Preferably, the ultimate degradation products are carbon dioxide, methane and water, although other degradation product may be perfectly accepted.

According to a preferred definition of biodegradability according to the invention, biodegradability is a property of certain organic molecules whereby, when exposed to the natural environment or placed within a living organism, they react through an enzymatic or microbial process, often in combination with a pure chemical process such as hydrolysis, to form simpler compounds, and ultimately, carbon dioxide, nitrogen oxides, methane and water.

Accordingly, suitable examples of additional environmentally or biologically degradable chewing gum base polymers, which can be applied in accordance with the gum base of the present invention, include degradable polyesters, poly(ester-carbonates), polycarbonates, polyester amides, polypeptides, homopolymers of amino acids such as polylysine, and proteins including derivatives thereof such as e.g. protein hydrolysates including a zein hydrolysate. Particularly useful compounds of this type include polyester polymers obtained by the polymerisation of one or more cyclic esters such as lactide, glycolide, trimethylene carbonate, δ-valerolactone, β-propiolactone and ε-caprolactone, and polyesters obtained by polycondensation of a mixture of open-chain polyacids and polyols, for example, adipic acid and di(ethylene glycol). Hydroxy carboxylic acids such as 6-hydroxycaproic acid may also be used to form polyesters or they may be used in conjunction with mixtures of polyacids and polyols. Such degradable polymers may be homopolymers, copolymers or terpolymers, including graft- and block-polymers.

According to further embodiments of the invention conventional elastomeric and/or resinous gum base compounds may be added to one or more of the chewing gum modules.

These include, but are not limited to synthetic elastomers listed in Food and Drug Administration, CFR, Title 21, Section 172,615, the Masticatory Substances, Synthetic) such as polyisobutylene. e.g. having a gas pressure chromatography (GPC) average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3:1, polyvinyl acetate (PVA), e.g. having a GPC average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer, and combinations hereof.

It is common in the industry to combine in a gum base a synthetic elastomer having a high molecular weight and a low molecular weight elastomer. Presently preferred combinations of synthetic elastomers include, but are not limited to, polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

In accordance with the invention, the chewing gum base components, which are used herein, may include one or more resinous compounds contributing to obtain the desired masticatory properties and acting as plasticizers for the elastomers of the gum base composition. In the present context, useful elastomer plasticizers include, but are not limited to, natural rosin esters, often referred to as ester gums including as examples glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins. Other useful resinous compounds include synthetic resins such as terpene resins derived from alpha-pinene, beta-pinene, and/or d-limonene, natural terpene resins; and any suitable combinations of the foregoing. The choice of elastomer plasticizers will vary depending on the specific application, and on the type of elastomer(s) being used.

A chewing gum base formulation may, if desired, include one or more fillers/texturisers including as examples, magnesium and calcium carbonate, sodium sulphate, ground limestone, silicate compounds such as magnesium and aluminum silicate, kaolin and clay, aluminum oxide, silicium oxide, talc, titanium oxide, mono-, di- and tri-calcium phosphates, cellulose polymers, such as wood, and combinations thereof.

The fillers/texturisers may also include natural organic fibers such as fruit vegetable fibers, grain, rice, cellulose and combinations thereof.

A gum base formulation may, in accordance with the present invention comprise one or more softeners e.g. sucrose polyesters including those disclosed in WO 00/25598, which is incorporated herein by reference, tallow, hydrogenated fat including tallow, hydrogenated and partially hydrogenated vegetable oils, cocoa butter, glycerol monostearate, glycerol triacetate, lecithin, mono-, di- and triglycerides, acetylated monoglycerides, fatty acids (e.g. stearic, palmitic, oleic and linoleic acids), and combinations thereof. As used herein the term "softener" designates an ingredient, which softens the gum base or chewing gum formulation and encompasses waxes, fats, oils, emulsifiers, surfactants and solubilisers.

To soften the gum base further and to provide it with water binding properties, which confer to the gum base a pleasant smooth surface and reduce its adhesive properties, one or more emulsifiers is/are usually added to the composition, typically in an amount of 0 to 18% by weight, preferably 0 to 12% by weight of the gum base. Mono- and diglycerides of edible fatty acids, lactic acid esters and acetic acid esters of mono and diglycerides of edible fatty acids, acetylated mono and diglycerides, sugar esters of edible fatty acids, Na-, K-, Mg- and Ca-stearates, lecithin, hydroxylated lecithin and the like are examples of conventionally used emulsifiers which can be added to the chewing gum base. In case of the presence of a biologically or pharmaceutically active ingredient as defined below, the formulation may comprise certain specific emulsifiers and/or solubilisers in order to enhance dispersion and release of the active ingredient.

Waxes and fats are conventionally used for the adjustment of the consistency and for softening of the chewing gum base when preparing chewing gum bases. In connection with the present invention, any conventionally used and suitable type of wax and fat may be used, such as for instance rice bran wax, polyethylene wax, petroleum wax (refined paraffin and microcrystalline wax), paraffin, beeswax, carnauba wax, candelilla wax, cocoa butter, degreased cocoa powder and any suitable oil or fat, as e.g. completely or partially hydrogenated vegetable oils or completely or partially hydrogenated animal fats.

Furthermore, the gum base formulation may, in accordance with the present invention, comprise colorants and whiteners such as FD&C-type dyes and lakes, fruit and vegetable extracts, titanium dioxide and combinations thereof. Further useful chewing gum base components include antioxidants, e.g. butylated hydroxytoluene (BHT), butyl hydroxyanisol (BHA), propylgallate and tocopherols, and preservatives.

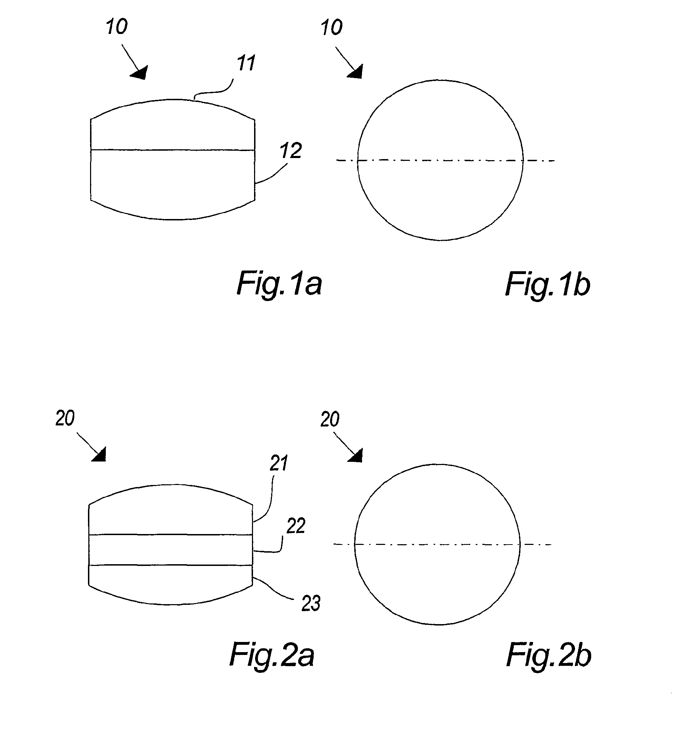

Different embodiments of the present invention are described by the figures 1-8, which are explained here below. The mechanical process of manufacturing some of these embodiments according to the present invention may be carried out as described in GB 1484832, hereby incorporated by reference. GB 1484832 discloses compression techniques using tableting machines and incorporating a minor part of plastic material. It should be noted, however, that GB 1484832 does not disclose chewing gum, as it does not involve any considerations about texture.

Fig. 1 a illustrates a cross-section of a compressed multi modular chewing gum tablet according the invention and illustrated in fig. 1b from above.

Generally, all of the below presented embodiments of the invention comprises at least one chewing gum module comprising biodegradable polymers unless otherwise stated and preferably, the illustrated embodiments solely comprise biodegradable gum base polymers.

Suitable biodegradable polymers of the below examples may be referred to as polyesters of type 1 and 2, which are described in PCT/DK03/00626, hereby incorporated by reference. Further descriptions of biodegradable polymers are disclosed in PCT/DK02/00201, PCT/DK02/00203, PCT/DK02/00205, PCT/DK02/ 00628 and PCT/DK03/00941, hereby incorporated by reference.

The illustrated chewing gum tablet 10 comprises two chewing gum modules 11 and 12.

According to the illustrated embodiment, each module is simply comprised by a layer. The multi-module tablet may in this embodiment be regarded as a two-layer chewing gum tablet 10.

The illustrated chewing gum tablet 10 may for example weight approximately 1.5 gram and comprise a non-GB chewing gum module 11 and a GB-containing module 12 (GB: gum base).

The illustrated non-GB chewing gum module 11 weights approximately 0.2 gram and the gum base-containing module 12 weights approximately 1.3 gram.

The illustrated tablet has an approximate diameter of 16 mm and a thickness at the thickest point in the center of approximately 7 mm.

Chewing gum module 12, here forming the gum base carrying part of the chewing gum, may comprise
a 16% gum base premix (comprising 12% menthol and 88% gum base),
57,4% sorbitol powder,
1% beads,
0,15% aspartame,
0,15% acesulfame,
1,3% peppermint powder and
24% gum base.

The biodegradable gum base may for example comprise

| | |
|---|---|
| Polyester type 1 elastomer: | 33.5 % by weight |
| Polyester type 2 resin: | 53.5 % by weight |
| Filler: | 5.0 % by weight |
| Emulsifier: | 2.0 % by weight |
| Fat | 2.0 % by weight |
| Wax: | 4.0 % by weight |

Chewing gum module 11 comprises
85% sorbitol
3% menthol powder,
2% eucalyptus powder
10% liquorice powder

The two modules 11 and 12 are adhered to each other. Different processes may be applied for the purpose. However, according to a preferred embodiment of the invention, the mutual adhering between the two layers is obtained by the compression of one module 11 onto the other 12.

According to an embodiment of the invention, the illustrated chewing gum tablet 10 may be provided with a coating, e.g. a film coating.

It should be noted that various concentrations of gum base in the different modules (here: layers) may be applied within the scope of the invention. Moreover, it should be noted that according to a preferred embodiment of the invention, the non-GB incorporated chewing gum layer should be substantially free of any gum base, i.e. as described above.

The non-GB (or little GB) incorporated modules may for instance comprise compressible chewing gum ingredients, for example sweeteners and flavors, more or less pre-processed for the purpose of facilitating a true compression. If, the non- or low GB-incorporated layer(s) has to include non-compressible ingredients, these may e.g. be incorporated in compressible materials or processed by known techniques.

Other optional ingredients to be emphasized here may e.g. comprise pharmaceutical ingredients.

In other applications, e.g. for the purpose of establishing different release profiles the different modules may comprise different amounts (i.e. concentrations) of gum base.

The tablet may moreover comprise (not shown) one or several barrier layers adapted for establishment of a barrier between inter-reacting ingredients and compounds, such as certain acids, flavors, active ingredients, polymer degradation enhancing compounds and/or biodegradable polymers.

Fig. 2a illustrates a cross-section of a compressed multi modular chewing gum tablet according to the invention and illustrated in fig. 2b from above.

The illustrated embodiment 20 comprises a three-module chewing gum of which the lowest layer 23 comprises a gum base incorporated chewing gum module having a certain gum base concentration, the intermediate layer 22 comprises a gum base incorporated chewing gum module of a gum base concentration differing from that of module 23 and the last module 21 comprises a substantially gum base-free chewing gum module.

The non-GB incorporated chewing gum module 21 may for example comprise compressed chewing gum ingredients, such as sweeteners, flavor, freeze-dried fruit etc. or a layer 11 as described in fig. 1a.

The two GB-containing modules 22 and 23 may for example comprise different concentrations of gum base, e.g. for the purpose of providing a variation, especially of the post release, whereas the module 21 primarily determines the initial release of the tablet when chewed.

Ingredients from module 21 may thus be chewed into module 22 and 23 during use, giving both a fast initial release more over serve as a post-mixing process whereby the ingredients of module 21 are mixed into the gum base containing modules. This is in particular useful for obtaining a combined fast release and slow release.

If the ingredients of 21 moreover comprise degradation enhancing compounds, these ingredients may be mixed with the biodegradable polymers of modules 22 and 23, thereby facilitating an increased degradation rate of the applied biodegradable polymers during and in particular after chew.

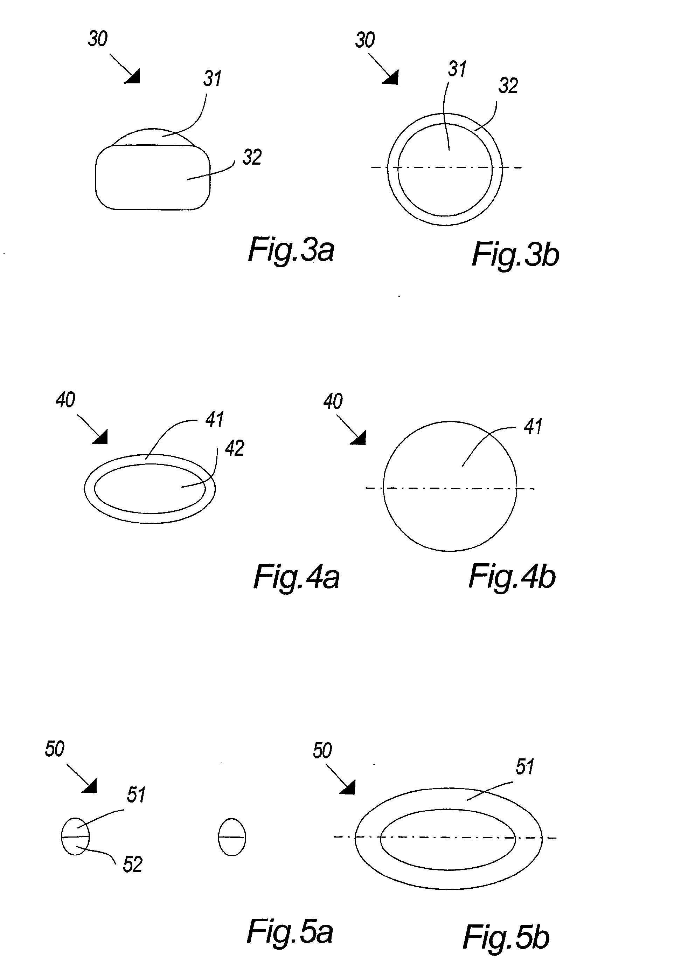

Fig. 3a illustrates a cross-section of a compressed multi modular chewing gum tablet 30 according the invention and illustrated in fig. 3b from above.

The illustrated chewing gum tablet 30 comprises a gum base incorporated chewing gum module 32 upon which a non-GB incorporated chewing gum base is arranged.

Fig. 4a illustrates a cross-section of a further compressed multi-modular chewing gum tablet 40 according to the invention and illustrated in fig. 4b from above.

The tablet 40 differs somewhat from the other described tablets in the sense that the tablet comprises a compressed GB-incorporated chewing gum module 42 forming a gum center. The module 42 is encapsulated by a surrounding substantially non-GB incorporated module 41.

Fig. 5a illustrates a cross-section of a compressed multi-modular chewing gum tablet 50 according to the invention and illustrated in fig. 5b from above.

According to the illustrated embodiment, showing a ring-formed two layer tablet 50, a chewing gum module 52 comprises a certain concentration of gum base, whereas the other layer comprises a non-gum base comprising module 51.

Alternatively, the chewing gum module 51 may comprise a gum base content differing from that of the chewing gum module 52, thereby facilitating a chewing gum providing at least two different release profiles in one piece.

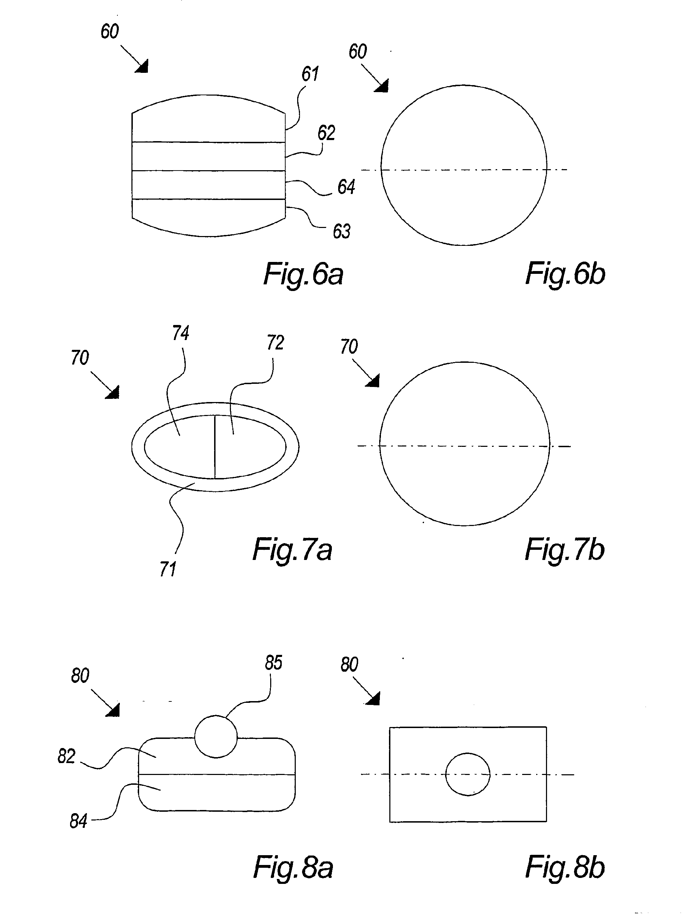

Fig. 6a illustrates a cross-section of a compressed multi-modular chewing gum tablet according to the invention and illustrated in fig. 6b from above.

The illustrated embodiment 60 comprises a four-module chewing gum in which one of the modules comprises conventional non-biodegradable gum base, which for example comprise

| | |
|---|---|
| elastomer: | 19 % by weight |
| natural resin: | 20 % by weight |
| synthetic resin: | 20 % by weight |
| fat/fillers: | 26 % by weight |
| wax: | 15 % by weight |

The non-biodegradable gum base may for example be comprised in chewing gum module 62, while the top module 61 and bottom module 64 may comprise substantially gum base free chewing gum modules and the intermediate chewing gum module 63 comprises biodegradable gum base according to the present invention.

It is thus noted according to the illustrated embodiment that a compressed chewing gum tablet may comprise different chewing gum modules and that these different modules may differ in weight, size and composition. Specifically, the above illustrated embodiment comprises a tablet wherein on of the modules basically comprises biodegradable gum base granules, namely module 63 and where another individual module may be based on conventional gum base polymers, namely module 62.

Such variation of the composition of the individual modules may generally facilitate an adjusted release profile due to the fact that different polymers release differently.

This may also be facilitated if all of the gum base containing modules comprises mutually different biodegradable polymers thereby rendering the complete tablet biodegradable.

Fig. 7a and 7b illustrates a further embodiment of a modular compressed chewing gum tablet 70 according to an embodiment of the invention.

It is noted that the "center" of the illustrated tablet comprises two modules, 72, 74 surrounded by a module 71. The outer module 71 may advantageously comprise a coating, which may be established in a conventional way or e.g. by means of compression.

In the illustrated exemplary chewing gum tablet 70, the two modules both comprise biodegradable polymer based gum base granules.

Fig. 8a and 8b illustrates a further embodiment of a modular compressed chewing gum tablet 80 according to an embodiment of the invention.

This tablet 80 basically comprises two individual modules 82, 84 and the tablet furthermore comprises a ball-shaped module 85.

In the illustrated exemplary chewing gum tablet 80, all three modules comprise biodegradable polymer based gum base granules.

### THE TABLET

The size of the tablet and individual tablets may vary significantly from tablet to tablet.

An example of a tablet (1.1 gram) may thus be 17mm x 7mm x 8 mm.

Another size and shape may be a round tablet (1.5 gram) having a diameter of 16mm, a thickness of 7.1 mm in the center a circumpheral thickness of approximately 4.1 mm

The tablets and the modules may have many different shapes. The preferred shape is the shape illustrated in fig. 1a and fig. 1b, i.e. a slice-like module. The modular shape is preferred due to the fact that it is relatively easy to handle and process. However, other module shapes may of course be applied within the scope of the invention.

A few of those are illustrated in fig. 3a, 3b, 4a, 4b, 5a, 5b, 6a, 6b, 7a, 7b, 8a and 8b.

## Claims

1. Chewing gum tablet comprising at least two individual coherent chewing gum modules,
at least one of said chewing gum modules comprising compressed gum base granules and wherein
said compressed gum base granules comprise at least one biodegradable polyester polymer.

2. Chewing gum tablet according to claim 1, wherein substantially all the chewing gum polymers are biodegradable.

3. Chewing gum tablet according to claims 1 or 2, wherein at least one biodegradable polymer in the amount of about 1% to about 100% by weight of the gum base granules.

4. Chewing gum tablet according to any of the claims 1-3, wherein at least one of said biodegradable polymers comprises a polyester produced through reaction of at least one alcohol or derivative thereof and at least one acid or derivative thereof.

5. Chewing gum tablet according to any of the claims 1-4,
wherein said alcohol derivative comprises an ester of an alcohol.

6. Chewing gum tablet according to any of the claims 1-5,
wherein at least one of said biodegradable polymers comprises a polymer obtained by polymerization of a at least one cyclic ester.

7. Chewing gum tablet according to any of the claims 1-6,
wherein said chewing gum comprises at least two different polymers.

8. Chewing gum tablet according to any of the claims 1-7, the chewing gum tablet comprises a gum base content of at least 5 % by weight of the tablet.

9. Chewing gum tablet according to any of the claims 1-8, wherein the chewing gum tablet (10, 20, 30, 40, 50) comprises a gum base content of at least 10 % by weight, preferably at least 15% by weight of the tablet.

10. Chewing gum tablet according to any of the claims 1-9, wherein said chewing gum comprises at least two chewing modules having different concentrations or composition of gum base.

11. Chewing gum tablet according to any of the claims 1-10, wherein said chewing gum tablet comprises at least one biodegradation enhancing compound.

12. Chewing gum tablet according to any of the claims 1-11, wherein said at least one biodegradation enhancing compounds comprises enzymes.

13. Chewing gum tablet according to any of the claims 1-12, wherein said biodegradation enhancing compounds are incorporated in at least one gum base containing module.

14. Chewing gum tablet according to any of the claims 1-13, wherein at least one of the chewing gum modules (11,21,31,41) has a gum base content of less than 5 % by weight.

15. Chewing gum tablet according to any of the claims 1-14, wherein at least one of the chewing gum modules (11,21,31,41) is substantially gum base free.

16. Chewing gum tablet according to any of the claims 1-15, wherein said substantially gum base free chewing gum comprises sweetener as the major ingredient

17. Chewing gum tablet according to any of the claims 1-16, wherein said chewing gum module comprising sweetener as the major ingredient forms a coating of the chewing gum tablet encapsulating the tablet completely or partly.

18. Chewing gum tablet according to any of the claims 1-17, wherein said biodegradation
enhancing compounds are incorporated in at least one substantially gum base-free module separated from said at least one module comprising biodegradable polymers.

19. Chewing gum tablet according to any of the claims 1-18, wherein all the chewing gum modules are made by compression.

20. Chewing gum tablet according to any of the claims 1-19, wherein the chewing gum modules are gathered by means of compression.

21. Chewing gum tablet according to any of the claims 1-20, whereby at least two, preferably all modules are compressed and gathered in one step.

22. Chewing gum tablet according to any of the claims 1-21, wherein said chewing gum modules formations having different concentrations or composition of chewing gum ingredients.

23. Chewing gum tablet according to any of the claims 1-22, wherein said modules are tablet slice-like layers.

24. Chewing gum tablet according to any of the claims 1-23, wherein different chewing gum modules comprises ingredients intended to be separated in the tablet.

25. Chewing gum tablet according to any of the claims 1-24, wherein at least two of said chewing gum modules are separated by at least one separation layer.

26. Chewing gum tablet according to any of the claims 1-25, wherein the thickness of at of at least one of said substantially gum base free layers exceeds at least the smallest width of the tablet divided by 20 (twenty).

27. Chewing gum tablet according to any of the claims 1-26, wherein the thickness of at of at least one of said substantially gum base free layers exceeds 0.5 mm, preferably 0.7 mm.

28. Chewing gum tablet according to any of the claims 1-27, wherein said chewing gum modules are manufactured on the basis of compressible chewing gum components.

29. Chewing gum tablet according to any of the claims 1-28, wherein said chewing gum comprises a coating.

30. Chewing gum tablet according to any of the claims 1-29, wherein the gum base has a water content of less than 1.0%, preferably substantially 0% by weight of the gum base.

31. Chewing gum tablet according to any of the claims 1-30, wherein the size of the gum base granules are within the range of 0.01mm 0.01 mm to 2mm 2mm, preferably within the range of 0.1mm 0.1mm to 1.0 mm 1.0mm previous to compression.

32. Method of manufacturing a chewing gum tablet according to any of the claims 1-31, whereby at least one of the chewing gum modules comprises active ingredients and thereby avoiding physical or chemical interaction between the chewing gum modules of the tablet.

## Patentansprüche

1. Kaugummitablette, umfassend mindestens zwei einzelne zusammenhängende Kaugummi-Module,
wobei mindestens eines der Kaugummi-Module komprimierte Gummibasis-Granula umfasst und wobei die komprimierten Gummibasis-Granula mindestens ein biologisch abbaubares Polyester-Polymer umfassen.

2. Kaugummitablette nach Anspruch 1, bei der im Wesentlichen alle Kaugummi-Polymere biologisch abbaubar sind.

3. Kaugummitablette nach Anspruch 1 oder 2, bei der mindestens ein biologisch abbaubares Polymer in einer Menge von etwa 1 bis etwa 100 Gew.-% der Gummibasis-Granula vorliegt.

4. Kaugummitablette nach irgendeinem der Ansprüche 1-3, bei der mindestens eines der biologisch abbaubaren Polymere einen Polyester umfasst, der durch Umsetzung mindestens eines Alkohols oder seines Derivats und mindestens einer Säure oder ihres Derivats hergestellt ist.

5. Kaugummitablette nach irgendeinem der Ansprüche 1-4, bei der das Alkohol-Derivat einen Ester eines Alkohols umfasst.

6. Kaugummitablette nach irgendeinem der Ansprüche 1-5, bei der mindestens eines der biologisch abbaubaren Polymere ein Polymer umfasst, das durch Polymerisation mindestens eines cyclischen Esters erhalten wurde.

7. Kaugummitablette nach irgendeinem der Ansprüche 1-6, bei der der Kaugummi mindestens zwei verschiedene Polymere umfasst.

8. Kaugummitablette nach irgendeinem der Ansprüche 1-7, bei der die Kaugummitablette einen Gummibasis-Gehalt von mindestens 5 Gew.-% der Tablette umfasst.

9. Kaugummitablette nach irgendeinem der Ansprüche 1-8, bei der die Kaugummitablette (10, 20, 30, 40, 50) einen Gummibasis-Gehalt von mindestens 10 Gew.-%, bevorzugt mindestens 15 Gew.-% der Tablette umfasst.

10. Kaugummitablette nach irgendeinem der Ansprüche 1-9, bei der der Kaugummi mindestens zwei Kau-Module mit (einer) verschiedenen Konzentrationen oder Zusammensetzung der Gummibasis umfasst.

11. Kaugummitablette nach irgendeinem der Ansprüche 1-10, bei der die Kaugummitablette mindestens eine den biologischen Abbau verstärkende Verbindung umfasst.

12. Kaugummitablette nach irgendeinem der Ansprüche 1-11, bei der mindestens eine der den biologischen Abbau verstärkenden Verbindungen Enzyme umfasst.

13. Kaugummitablette nach irgendeinem der Ansprüche 1-12, bei der die den biologischen Abbau verstärkenden Verbindungen in mindestens einem Gummibasis-haltigen Modul enthalten sind.

14. Kaugummitablette nach irgendeinem der Ansprüche 1-13, bei der mindestens eines der Kaugummi-Module (11, 21, 31, 41) einen Gummibasis-Gehalt von weniger als 5 Gew.-% aufweist.

15. Kaugummitablette nach irgendeinem der Ansprüche 1-14, bei der mindestens eines der Kaugummi-Module (11, 21, 31, 41) im Wesentlichen frei von Gummibasis ist.

16. Kaugummitablette nach irgendeinem der Ansprüche 1-15, bei der der im Wesentlichen Gummibasis-freie Kaugummi Süßungsmittel als Hauptbestandteil umfasst.

17. Kaugummitablette nach irgendeinem der Ansprüche 1-16, bei der das Kaugummi-Modul, das Süßungsmittel als Hauptbestandteil umfasst, eine Beschichtung der Kaugummitablette bildet, welche die Tablette vollständig oder teilweise einkapselt.

18. Kaugummitablette nach irgendeinem der Ansprüche 1-17, bei der die den biologischen Abbau verstärkenden Verbindungen in mindestens einem im Wesentlichen Kaugummibasis-freien Modul enthalten sind, welches von dem mindestens einen Modul, das biologisch abbaubare Polymere umfasst, getrennt vorliegt.

19. Kaugummitablette nach irgendeinem der Ansprüche 1-18, bei der alle Kaugummi-Module durch Kompression hergestellt sind.

20. Kaugummitablette nach irgendeinem der Ansprüche 1-19, bei der die Kaugummi-Module mittels Kompression zusammengefügt sind.

21. Kaugummitablette nach irgendeinem der Ansprüche 1-20, bei der mindestens zwei, bevorzugt alle Module in einem Schritt komprimiert und zusammengefasst werden.

22. Kaugummitablette nach irgendeinem der Ansprüche 1-21, bei der die Kaugummi-Modulgebilde verschiedene Konzentrationen oder eine verschiedene Zusammensetzung an Kaugummi-Bestandteilen aufweisen.

23. Kaugummitablette nach irgendeinem der Ansprüche 1-22, bei der die Module scheibenartige Tablettenschichten sind.

24. Kaugummitablette nach irgendeinem der Ansprüche 1-23, bei der verschiedene Kaugummi-Module Bestandteile umfassen, die in der Tablette getrennt vorliegen sollen,

25. Kaugummitablette nach irgendeinem der Ansprüche 1-24, bei der mindestens zwei der Kaugummi-Module durch mindestens eine Trennschicht getrennt sind.

26. Kaugummitablette nach irgendeinem der Ansprüche 1-25, bei der die Dicke mindestens eine der im Wesentlichen Gummibasis-freien Schichten mindestens die geringste Breite der Tablette dividiert durch 20 (zwanzig) überschreitet.

27. Kaugummitablette nach irgendeinem der Ansprüche 1-26, bei der die Dicke mindestens einer der im Wesentlichen Gummibasis-freien Schichten 0,5 mm, vorzugsweise 0,7 mm überschreitet.

28. Kaugummitablette nach irgendeinem der Ansprüche 1-27, bei der die Kaugummi-Module auf der Basis von komprimierbaren Kaugummi-Komponenten hergestellt werden.

29. Kaugummitablette nach irgendeinem der Ansprüche 1-28, bei der der Kaugummi eine Beschichtung umfasst.

30. Kaugummitablette nach irgendeinem der Ansprüche 1-29, bei der die Gummibasis einen Wassergehalt von weniger als 1,0, bevorzugt im Wesentlichen 0 Gew.-% der Gummibasis aufweist.

31. Kaugummitablette nach irgendeinem der Ansprüche 1-30, bei der die Größe der Gummibasis-Granula vor der Kompression im Bereich von 0,01 mm • 0,01 mm bis 2 mm 2 mm, bevorzugt im Bereich von 0,1 mm • 0,1 mm bis 1,0 mm • 1,0 mm liegt.

32. Verfahren zur Herstellung einer Kaugummitablette nach irgendeinem der Ansprüche 1-31, bei dem mindestens eines der Kaugummi-Module aktive Bestandteile umfasst und bei dem eine physikalische oder chemische Wechselwirkung zwischen den Kaugummi-Modulen der Tablette vermieden wird.

## Revendications

1. Pastille de gomme à mâcher comprenant au moins deux modules cohérents individuels de gomme à mâcher, au moins un desdits modules de gomme à mâcher comprenant des granules compressées de base de gomme et dans lesquelles lesdites granules compressées de base de gomme comprennent au moins un polymère de polyester biodégradable.

2. Pastille de gomme à mâcher selon la revendication 1, dans laquelle pratiquement la totalité des polymères de gomme à mâcher sont biodégradables.

3. Pastille de gomme à mâcher selon la revendication 1 ou 2, dans laquelle on trouve au moins un polymère biodégradable en une quantité d'environ 1 % à 100 % en poids des granules de base de gomme.

4. Pastille de gomme à mâcher selon l'une quelconque des revendications 1 à 3, dans laquelle au moins un desdits polymères biodégradables comprend un polyester produit par la réaction d'au moins un alcool ou d'un dérivé de celui-ci et d'au moins un acide ou d'un dérivé de celui-ci.

5. Pastille de gomme à mâcher selon l'une quelconque des revendications 1 à 4, dans laquelle ledit dérivé d'alcool comprend un ester d'alcool.

6. Pastille de gomme à mâcher selon l'une quelconque des revendications 1 à 5, dans laquelle au moins un desdits polymères biodégradables comprend un polymère obtenu par la polymérisation d'au moins un ester cyclique.

7. Pastille de gomme à mâcher selon l'une quelconque des revendications 1 à 6, dans laquelle ladite gomme à mâcher comprend au moins deux polymères différents.

8. Pastille de gomme à mâcher selon l'une quelconque des revendications 1 à 7, la pastille de gomme à mâcher comprenant une teneur en base de gomme d'au moins 5 % en poids de la pastille.

9. Pastille de gomme à mâcher selon l'une quelconque des revendications 1 à 8, dans laquelle la pastille de gomme à mâcher (10, 20, 30, 40, 50) comprend une teneur en base de gomme d'au moins 10 % en poids, de préférence d'au moins 15 % en poids de la pastille.

10. Pastille de gomme à mâcher selon l'une quelconque des revendications 1 à 9, dans laquelle ladite gomme à mâcher comprend au moins deux modules à mâcher ayant différentes concentrations ou compositions de gomme de base.

11. Pastille de gomme à mâcher selon l'une quelconque des revendications 1 à 10, dans laquelle ladite pastille de gomme à mâcher comprend au moins un composé renforçant la biodégradation.

12. Pastille de gomme à mâcher selon l'une quelconque des revendications 1 à 11, dans laquelle ledit au moins un composé renforçant la biodégradation comprend des enzymes.

13. Pastille de gomme à mâcher selon l'une quelconque des revendications 1 à 12, dans laquelle lesdits composés renforçant la biodégradation sont incorporés dans au moins un module contenant une base de gomme.

14. Pastille de gomme à mâcher selon l'une quelconque des revendications 1 à 13, dans laquelle au moins un des modules de gomme à mâcher (11, 21, 31, 41) possède une teneur en base de gamme inférieure à 5 % en poids.

15. Pastille de gomme à mâcher selon l'une quelconque des revendications 1 à 14, dans laquelle au moins un des modules de gomme à mâcher (11, 21, 31, 41) est sensiblement exempt de base de gomme.

16. Pastille de gomme à mâcher selon l'une quelconque des revendications 1 à 15, dans laquelle ladite gomme à mâcher sensiblement exempte de base de gomme comprend un édulcorant en tant qu'ingrédient principal.

17. Pastille de gomme à mâcher selon l'une quelconque des revendications 1 à 16, dans laquelle ledit module de gomme à mâcher comprenant un édulcorant en tant qu'ingrédient principal forme un enrobage sur la pastille de gomme à mâcher encapsulant complètement ou partiellement la pastille.

18. Pastille de gomme à mâcher selon l'une quelconque des revendications 1 à 17, dans laquelle les composés renforçant la biodégradation sont incorporés dans au moins un module sensiblement exempt de base de gomme sépare dudit au moins un module comprenant des polymères biodégradables.

19. Pastille de gomme à mâcher selon l'une quelconque des revendications 1 à 18, dans laquelle tous les modules de gomme à mâcher sont préparés par compression.

20. Pastille de gomme à mâcher selon l'une quelconque des revendications 1 à 19, dans laquelle les modules de gomme à mâcher sont réunis par compression.

21. Pastille de gomme à mâcher selon l'une quelconque des revendications 1 à 20, moyennant quoi au moins deux modules, de préférence tous les modules, sont comprimés et réunis en une étape.

22. Pastille de gomme à mâcher selon l'une quelconque des revendications 1 à 21, dans laquelle les formations desdits modules de gomme à mâcher possèdent différentes concentrations ou compositions en ingrédients de gomme à mâcher.

23. Pastille de gomme à mâcher selon l'une quelconque des revendications 1 à 22, dans laquelle lesdits modules sont des couches de type tranche de pastille.

24. Pastille de gomme à mâcher selon l'une quelconque des revendications 1 à 23, dans laquelle les différents modules de gomme à mâcher comprennent des ingrédients destinés à être séparés dans la pastille.

25. Pastille de gomme à mâcher selon l'une quelconque des revendications 1 à 24, dans laquelle au moins deux desdits modules de gomme à mâcher sont séparés par au moins une couche de séparation.

26. Pastille de gomme à mâcher selon l'une quelconque des revendications 1 à 25, dans laquelle l'épaisseur d'au moins une desdites couches sensiblement exemptes de base de gomme dépasse au moins la largeur la plus petite de la pastille divisée par 20 (vingt).

27. Pastille de gomme à mâcher selon l'une quelconque des revendications 1 à 26, dans laquelle l'épaisseur d'au moins une desdites couches sensiblement exemptes de base de gomme dépasse 0,5 mm, de préférence 0,7 mm.

28. Pastille de gomme à mâcher selon l'une quelconque des revendications 1 à 27, dans laquelle lesdits modules de gomme à mâcher sont fabriqués à partir de composants de gomme à mâcher pouvant être compressés.

29. Pastille de gomme à mâcher selon l'une quelconque des revendications 1 à 28, dans laquelle ladite gomme à mâcher comprend un enrobage.

30. Pastille de gomme à mâcher selon l'une quelconque des revendications 1 à 29, dans laquelle la base de gomme possède une teneur en eau inférieure à 1,0 %, de préférence sensiblement égale à 0 % en poids de la base de gomme.

31. Pastille de gomme à mâcher selon l'une quelconque des revendications 1 à 30, dans laquelle la taille des granules de base de gomme sont dans la plage allant de 0,01 mm·0,01 mm à 2 mm·2 mm, de préférence dans la plage allant de 0,1mm·0,1 mm à 1,0 mm_{·}1,0 mm avant la compression.

32. Procédé de fabrication d'une pastille de gomme à mâcher selon l'une quelconque des revendications 1 à 31, moyennant quoi au moins un des modules de gomme à mâcher comprend des ingrédients actifs et ainsi une interaction physique ou chimique entre les modules de gomme à mâcher de la pastille est évitée.
